# EUROPÄISCHE PATENTSCHRIFT

(11) **EP 1 786 832 B1**
(45) Veröffentlichungstag und Bekanntmachung des Hinweises auf die Patenterteilung: **18.07.2012**
(21) Anmeldenummer: 05786305.2
(22) Anmeldetag: 06.09.2005
(51) Int. Cl.: C07K 14/33

(54) **TRANSPORTPROTEIN ZUM EINBRINGEN CHEMISCHER VERBINDUNGEN IN NERVENZELLEN**
TRANSPORT PROTEIN WHICH IS USED TO INTRODUCE CHEMICAL COMPOUNDS INTO NERVE CELLS
PROTEINE DE TRANSPORT POUR L'INTRODUCTION DE COMPOSES CHIMIQUES DANS DES CELLULES NERVEUSES

(30) Priorität: 06.09.2004 DE 102004043009
(43) Veröffentlichungstag der Anmeldung: 23.05.2007
(73) Patentinhaber: toxogen GmbH, 30625 Hannover (DE)
(72) Erfinder: RUMMEL, Andreas, 30625 Hannover (DE)
(74) Vertreter: Wilk, Thomas
(86) Internationale Anmeldenummer: PCT/EP2005/009554
(87) Internationale Veröffentlichungsnummer: WO 2006/027207

(56) Entgegenhaltungen:
- WO-A-00/55208
- IHARA H ET AL: "Sequence of the gene for Clostridium botulinum type B neurotoxin associated with infant botulism, expression of the C-terminal half of heavy chain and its binding activity" BIOCHIMICA ET BIOPHYSICA ACTA . GENE STRUCTURE AND EXPRESSION, ELSEVIER, AMSTERDAM, NL, Bd. 1625, Nr. 1, 3. Januar 2003 (2003-01-03), Seiten 19-26, XP004402176 ISSN: 0167-4781
- GINALSKI^A^ ^B K ET AL: "Structure-based sequence alignment for the beta-trefoil subdomain of the clostridial neurotoxin family provides residue level information about the putative ganglioside binding site" FEBS LETTERS, ELSEVIER, AMSTERDAM, NL, Bd. 482, Nr. 1-2, 29. September 2000 (2000-09-29), Seiten 119-124, XP004337632 ISSN: 0014-5793
- GOODNOUGH M C ET AL: "Development of a delivery vehicle for intracellular transport of botulinum neurotoxin antagonists" FEBS LETTERS, ELSEVIER, AMSTERDAM, NL, Bd. 513, Nr. 2-3, 27. Februar 2002 (2002-02-27), Seiten 163-168, XP004629890 ISSN: 0014-5793
- RUMMEL ANDREAS ET AL: "The Hcc-domain of botulinum neurotoxins A and B exhibits a singular ganglioside binding site displaying serotype specific carbohydrate interaction." MOLECULAR MICROBIOLOGY, Bd. 51, Nr. 3, Februar 2004 (2004-02), Seiten 631-643, XP002363868 ISSN: 0950-382X
- SUTTON J M ET AL: "Tyrosine-1290 of tetanus neurotoxin plays a key role in its binding to gangliosides and functional binding to neurones" FEBS LETTERS, ELSEVIER, AMSTERDAM, NL, Bd. 493, Nr. 1, 23. März 2001 (2001-03-23), Seiten 45-49, XP004257397 ISSN: 0014-5793
- HERREROS J ET AL: "C-terminal half of tetanus toxin fragment C is sufficient for neuronal binding and interaction with a putative protein receptor", BIOCHEMICAL JOURNAL, THE BIOCHEMICAL SOCIETY, LONDON, GB, vol. 347, no. 1, 1 April 2000 (2000-04-01) , pages 199-204, XP002173091, ISSN: 0264-6021, DOI: DOI:10.1042/0264-6021:3470199
- DATABASE BIOSIS [Online] BIOSCIENCES INFORMATION SERVICE, PHILADELPHIA, PA, US August 2008 STENMARK PAL ET AL: 'Crystal structure of botulinum neurotoxin type a in complex with the cell surface co-receptor GT1b - Insight into the toxin-neuron interaction' Database accession no. PREV200800648868 & PLOS PATHOGENS, vol. 4, no. 8, August 2008 (2008-08), pages Article No.: e1000129 URL-http://ww, ISSN: 1553-7366(print), DOI: DOI:10.1371/JOURNAL.PPAT.1000129

## Beschreibung

Die vorliegende Erfindung bezieht sich auf ein gemäβ den nachfolgenden Ansprüchen definiertes Transportprotein, das an Neurone bindet, durch rezeptorvermittelte Endozytose aufgenommen und aus dem sauren, endosomalen Kompartiment in das Zytosol von Neuronen transloziert wird. Dieses Protein findet Verwendung als Transporter, der andere chemische Substanzen (z.B. Proteasen) transloziert, die physiologisch nicht durch die Plasmamembran in das Zytosol von Nervenzellen eindringen können. Die vorliegende Erfindung bezieht sich auf die Verwendung eines Transportproteins zur Inhibition der Freisetzung von Neurotransmittern.

Nervenzellen setzen Transmitterstoffe durch Exozytose frei. Als Exozytose wird die Verschmelzung der Membranen intrazellulärer Vesikel mit der Plasmamembran bezeichnet. Bei diesem Vorgang wird gleichzeitig der vesikuläre Inhalt in den synaptischen Spalt ausgeschüttet. Die Fusion der beiden Membranen wird durch Calcium reguliert, welches mit dem Protein Synaptotagmin reagiert. Zusammen mit anderen Kofaktoren kontrolliert Synaptotagmin den Status von drei sogenannten Fusionsproteinen, dem SNAP-25, Synaptobrevin 2 und Syntaxin 1A. Während Syntaxin 1A und Synaptobrevin 2 in die Plasma- bzw. Vesikelmembran integriert sind, ist SNAP-25 nur schwach an die Plasmamembran gebunden. Bei einem Anstieg der intrazellulären Galcium-Konzentration binden die drei Proteine aneinander, wobei sich beide Membranen annähern und anschließend miteinander verschmelzen. Bei cholinergen Neuronen wird Acetylcholin freigesetzt, welches Muskelkontraktionen, Schweißabsonderung und andere cholinerg vermittelte Reaktionen verursacht.

Die oben genannten Fusionsproteine sind die Zielmoleküle (Substrate) der leichten Ketten der clostridiellen Neurotoxine, die vom Bakterium *Clostridium botulinum* gebildet werden.

Das anaerobe, gram-positive Bakterium *Clostridium botulinum* produziert sieben verschiedene Typen an Protein-Neurotoxinen. Diese werden als die Botulinus Neurotoxine (BoNT/A bis BoNT/G) bezeichnet. Hiervon verursachen insbesondere BoNT/A und BoNT/B bei Mensch und Tier eine neuroparalytische Erkrankung, die als Botulismus bezeichnet wird. Die Sporen von *Clostridium botulinum* finden sich im Erdreich, können sich jedoch in unsachgemäß sterilisierten und verschlossenen Nahrungsmittellconserven aus häuslicher Herstellung entwickeln, auf die viele der Botulismusfälle zurückgeführt werden.

BoNT/A ist die letalste aller bekannten biologischen Substanzen. Nur etwa 5-6 pg gereinigtes BoNT/A ist eine MLD (Multiple Low Dose). Eine Einheit (engl.: Unit, U) von BoNT wird als die MLD definiert, die nach intraperitonealer Injektion die Hälfte an weiblichen Swiss Webster-Mäusen im Gewicht von jeweils 18-20 g tötet. Sieben immunologisch unterschiedliche BoNT wurden charakterisiert. Sie tragen die Bezeichnungen BoNT/A, B, C₁, D, E, F und G und können durch Neutralisation mit Serotyp spezifischen Antikörpern unterschieden werden. Die verschiedenen Serotypen von BoNT unterscheiden sich bei betroffenen Tierarten hinsichtlich der Schwere und Dauer der verursachten Lähmung. So ist z.B. bei der Ratte im Hinblick auf die Lähmung BoNT/A 500mal stärker wirksam als BoNT/B. Hinzu kommt, dass BoNT/B sich bei Primaten in einer Dosierung von 480 U/kg Körpergewicht als untoxisch erwiesen hat. Dieselbe Menge BoNT/A entspricht der 12-fachen letalen Dosis (LD) dieses Stoffes bei Primaten. Zum anderen ist bei Mäusen die Lähmungsdauer nach Injektion von BoNT/A 10fach länger als nach Injektion von BoNT/E.

Die BoNT sind klinisch zur Behandlung neuromuskulärer Störungen eingesetzt worden, die durch Hyperaktivität in Skelettmuskeln, verursacht durch pathologisch überaktive periphere Nerven, charakterisiert sind. BoNT/A ist von der U.S. Food and Drug Administration zur Behandlung von Blepharospasmus, Strabismus und Hemifacialspasmen zugelassen. Verglichen mit BoNT/A besitzen die übrigen BoNT Serotypen offensichtlich eine geringere Wirksamkeit und kürzere Wirkdauer. Klinisch Effekte des peripher intramuskulär verabreichten BoNT/A stellen sich für gewöhnlich innerhalb einer Woche ein. Die Dauer der Symptomunterdrückung, durch eine einzige intramuskuläre Injektion von BoNT/A beläuft sich im Regelfall auf etwa 3 Monate.

Die clostridiellen Neurotoxine hydrolysieren spezifisch verschiedene Proteine des Fusionsapparates. BoNT/A, C₁ und E spalten SNAP-25, während BoNT/B, D, F, G sowie Tetanus Neurotoxin (TeNT) das vesikel-assoziierte Membranprotein (VAMP) 2 - auch Synaptobrevin 2 genannt - angreifen. BoNT/C₁ spaltet außerdem Syntaxin 1 A.

Die Clostridien Bakterien setzen die Neurotoxine als einkettige Polypeptide mit jeweils 1251 bis 1315 Aminosäuren frei. Nachfolgend spalten endogene Proteasen jedes dieser Proteine an einer bestimmten Stelle in jeweils 2 Ketten (,nicking'), wobei die beiden Ketten jedoch durch eine Disulfid-Brücke miteinander verbunden bleiben. Diese zweikettigen Proteine werden als Holotoxine bezeichnet (siehe Shone et al. (1985), Eur J Biochem 151, 75-82). Die beiden Ketten haben verschiedene Funktionen. Während das kleinere Teilstück, die leichte Kette (light chain = LC), eine Zn²⁺-abhängige Endoprotease darstellt, ist die größere Einheit (heavy chain = HC) der Transporter der leichten Kette. Durch Behandlung der HC mit Endopeptidasen ergaben sich zwei 50 kDa Fragmente (siehe Gimenez et al. (1993), J Protein Chem 12, 351-363). Die amino-terminale Hälfte (H_{N}-Fragment) integriert sich bei niedrigem pH-Wert in Membranen und verhilft der LC, in das Zytosol der Nervenzelle einzudringen. Die carboxy-terminale Hälfte (H_{C}-Fragment) bindet an komplexe Polysialoganglioside, die nur in Nervenzellmembranen vorkommen, und an bislang nicht identifizierte Proteinrezeptoren (Halpern et al. (1993), Curr Top Microbiol Immunol 195, 221-241). Dies erklärt die hohe Neuroselektivität der clostridiellen Neurotoxine. Kristallstrukturen bestätigen, dass BoNT/A über drei Domänen verfügt, die mit den drei Schritten des Wirkmechanismus in Einklang gebracht werden können (siehe Lacy et al. (1998), Nat Struct Biol 5, 898-902). Desweiteren lassen diese Daten darauf schließen, dass innerhalb des H_{C}-Fragments zwei autonome Untereinheiten (Subdomänen) von jeweils 25 kDa existieren. Der erste Beweis für die Existenz der beiden funktionellen Subdomänen wurde mit der amino-terminalen (H_{CN}) und der carboxyterminalen Hälfte (H_{CC}) des H_{C}-Fragments des TeNT erbracht, die rekombinant exprimiert wurden und erkennen liessen, dass zwar die H_{CC}-, nicht aber die H_{CN}-Domäne an Neurone bindet (siehe Herreros et al. (2000), Biochem J 347, 199-204). Die Proteinrezeptor-Bindungsstelle des Synaptotagmin wurde innerhalb der H_{CC}-Domänen von BoNT/B und G entdeckt, wodurch deren separate Funktionalität gezeigt wurde (siehe Rummel et al. (2004), J Biol Chem 279, 30865-70).

Unter physiologischen Bedingungen bindet die HC an neuronale Ganglioside, wird durch rezeptorvermittelte Endozytose ins Zellinnere aufgenommen und erreicht über das endosomale Kompartiment den natürlichen Vesikellcreislauf. Im sauren Milieu der frühen Endosomen dringt H_{N}, die amino-terminale Hälfte von HC, in die Vesikelmembran ein und bildet eine Pore. Jede Substanz (X), die mit HC über eine Disulfid-Brücke verbunden ist, wird durch intrazelluläre Redoxsysteme, die Zugang zur Disulfid-Brücke bekommen und sie reduzieren, von der HC getrennt werden. Letztendlich wird X im Zytosol erscheinen.

Im Falle der clostridiellen Neurotoxine ist die HC der Träger einer LC, die im finalen Schritt ihr spezifisches Substrat im Zytosol spaltet. Der Zyklus der Komplexbildung und -dissoziation der Fusionsproteine wird unterbrochen und somit die Ausschüttung von Acetylcholin gehemmt. Als Folge hiervon werden gestreifte Muskeln gelähmt, und Schweißdrüsen stellen ihre Sekretion ein. Die Wirkdauer der einzelnen BoNT Serotypen ist verschieden und hängt von der Präsenz intakter LC im Zytosol ab. Da alle Neurone Rezeptoren für clostridielle Neurotoxine besitzen, ist es nicht nur die Ausschüttung von Acetylcholin, die betroffen sein kann, sondern potentiell auch die Ausschüttung der Substanz P, von Noradrenalin, GABA, Glycin, Endorphin und anderer Transmitter und Hormone.

Dass bevorzugt die cholinerge Transmission blockiert wird, kann damit erklärt werden, dass die HC in der Peripherie in das Neuron eindringt. Zentrale Synapsen werden durch die Blut-Hirn-Schranke geschützt, die von Proteinen nicht überwunden werden kann.

Die HC besitzen eine hohe Affinität zu peripheren Nervenzellen, die überwiegend durch die Interaktion mit komplexen Polysialogangliosiden - dies sind Glycolipide, die aus mehr als einer Sialinsäure bestehen - vermittelt wird (siehe Halpern et al. (1995), Curr Top Micrbiol Immunol 195, 221-41). Folglich erreichen die an sie gebundenen LC nur diesen Zelltyp und werden nur in diesen Zellen wirksam. BoNT/A und B binden lediglich je ein Molekül Gangliosid GT1b.

Um die Rolle der Aminosäuren zu erforschen, welche die Bindungstasche aufbauen, wurde erfindungsgemäß rekombinantes H_{C}-Fragment hergestellt. Diese Technik gestattet es, einzelne Aminosäuren auszutauschen. So können positiv geladene Aminosäuren durch negativ geladene oder neutrale Aminosäuren ersetzt werden, und umgekehrt. Kleine Änderungen an der Oberfläche der Bindungstasche haben dramatische Wirkungen hinsichtlich der Passfähigkeit der Ganglioside. Es konnte gezeigt werden, dass die Affinität um mehr als 99% zurückging, wenn z.B. die Aminosäure in Position 1266, das Tryptophan - als W im SXWY- Motiv bezeichnet- gegen einen aliphatischen Rest, z.B. Leucin, ausgetauscht wird. Jedoch wurde auch Gegenteiliges beobachtet. Der Austausch von Aminosäuren, die in die Bindungstasche hineinreichen, führte zu einer Zunahme der Affinität zu Gangliosiden. Da die Gestalt der Bindungstasche so entscheidend für die Affinität der HC zum Gangliosidrezeptor ist, nimmt gleichzeitig mit der Affinität von HC zum Gangliosidrezeptor auch die proteolytische Potenz der anhängenden LC im Einklang mit der Affinität entweder zu oder ab.

In einer Ligand-Rezeptor Studie wurden daher erfindungsgemäß bestimmte Aminosäurenreste in der gangliosidbindenden Tasche von BoNT/A charakterisiert und ausgetauscht, um so die Affinität an den Gangliosidrezeptor zu erhöhen. Die Affinität des mutierten H_{C}-Fragments wurde in Gangliosid- und Synaptosomen-Bindungsassays bestimmt. Sodann wurde die HC mit denselben Mutationen an LC-A gekoppelt, wozu eine Thrombin sensitive Aminosäurensequenz benutzt wurde. Das rekombinante Protein wurde mit Thrombin aktiviert (,nicked') und führte zu einem zweikettigen Molekül, bei denen beide Ketten durch eine einzige Disulfid-Brücke miteinander verbunden waren. Die Wirkstärke der Konstrukte wurde an Synaptosomen aus Rattenhirn - einem Präparat, das Transmitter freisetzt - getestet. Das Ausmaß der Hemmung der Transmitterausschüttung galt als Gradmesser der Wirkstärke der Konstrukte. Zusätzlich wurde die Potenz der einzelnen Konstrukte mit Hilfe des isolierten Nerv-Muskel-Präparats der Maus (Hemi-Diaphragma-Assay = HDA), welches das physiologische Ziel der clostridiellen Neurotoxine darstellt, analysiert.

Erkrankungen und Symptome, die mit TrapoX behandelt werden sollen, gehen einher mit einer fokal erhöhten Aktivität von Motoneuronen und von vegetativen Nervenzellen. Die erhöhte Aktivität führt zu schmerzhaften Verkrampfungen der von diesen Zellen innervierten Muskeln und zu übermäßiger Flüssigkeitssekretion aus Drüsenzellen. Weiterhin treten durch die erhöhte Aktivität Gesichtsfalten in verschiedenen Regionen auf. Die Ursache ist eine pathologisch gesteigerte Freisetzung von Azetylcholin aus den peripheren Nervenendungen. Wird TrapoX in den betroffen Muskel injiziert, kommt es nach einer Latenzzeit von 1-3 Tagen zur Erschlaffung der betroffenen Muskeln, zum Versiegen der Sekretion und zur Glättung der Gesichtshaut. Dies ist bedingt durch eine Hemmung der Freisetzung von Azetylcholin durch TrapoX. Der Patient wird nahezu beschwerdefrei und die durch den Muskelkrampf ausgelösten Schmerzen lassen nach und verschwinden vollständig.

Die Freisetzung von Azetylcholin wird sowohl im Mensch als auch im Tier gehemmt. Daher wird sowohl zum Nachweis von BoNT bei Vergiftungen als auch zur Aktivitätsbestimmung von BoNT-Medikamenten (Botox, Dysport, Xeomin) routinemäßig der Tierversuch eingesetzt. Die Aktivität von BoNT wird quantifiziert, indem man eine Bestimmung der LD₅₀ in der Maus durchführt. Hierbei wird die Dosis gesucht, die 50% der Tiere aus einer Population tötet. Es ist offensichtlich, dass neben Dosen, die kein Tier töten, auch Dosen verabreicht werden, die 100% der Tiere einer Gruppe töten. Da das Gift systemisch (i.p.) gegeben wird, sterben also eine große Anzahl der Tiere qualvoll an einer Atemlähmung, ausgelöst durch eine Paralyse der Atemmuskulatur. Zur Vermeidung der Tierversuche wurde von uns der Maus Hemidiaphragma Assay eingeführt. Versuchsmäuse sterben im LD₅₀ Test an einer Atemlähmung, die durch Paralyse der Atemmuskulatur verursacht wird. Man kann also einer Maus den Atemmuskel inldusive des innervierenden Nervs (Nervus phrenicus) entnehmen und in vitro vergiften. BoNT wird an seine Rezeptoren binden, in die Zelle aufgenommen und transloziert werden und schließlich sein Substrat spalten, woraufhin der Muskel paralysiert. Es gibt eine strenge Korrelation zwischen dem LD₅₀ Wert und der Paralyse des Atemmuskels. Dieser in vitro Test stellt gewissermaßen eine abgespeckte Version des Tierversuchs dar (Wohlfarth K, Goeschel H, Frevert J, Dengler R, Bigalke H, Botolinum A toxis: units versus units. Naunyn Schmiedebergs Arch Phamacol. 1997 Mar;335(3):335-40).

Man kann also davon ausgehen, dass das BoNT, welches das Zwerchfell in vitro paralysiert, auch in der lebenden Maus wirkt und sie entsprechend der verabreichten Dosis auch tötet. Diese Tierversuchsersatzmethode ist so überzeugend, dass der Maus Hemidiaphragma Assay in Kürze von der EU Pharmakopöe als offizielle behördliche Prüfmethode auf BoNT für die EU-Mitgliedstaaten akzeptiert wird. Wir können daher von der erhöhten Wirksamkeit von TrapoX am Maus-Zwerchfell-Präparat auf eine erhöhte Wirksamkeit auch im Menschen schließen.

Der BoNT/A-Komplex wurde in der jüngeren Vergangenheit zur Behandlung motorischer Dystonien eingesetzt, sowie zur Dämpfung exzessiver sympathischer Aktivität (siehe Benecke et al. (1995), Akt Neurol 22, 209ff) und zur Linderung von Schmerz und Migräne (siehe Sycha et al. (2004), J Neurol 251, 19-30). Dieser Komplex besteht aus dem Neurotoxin, verschiedenen Hämagglutininen und einem nicht toxischen, nicht hämagglutinierenden Protein. Unter physiologischem pH dissoziiert der Komplex schnell. Das hieraus hervorgehende Neurotoxin ist der einzige Bestandteil des Komplexes, der therapeutisch relevant ist und eine Linderung der Symptome verursacht. Da die zugrundeliegende neurologische Erkrankung nicht geheilt wird, muss der Komplex in Intervallen von drei bis vier Monaten erneut injiziert werden. In Abhängigkeit von der Menge des injizierten Fremdproteins bilden einige Patienten spezifische BoNT/A-Antikörper. Diese Patienten werden gegen das Neurotoxin resistent. Wenn erst einmal antigensensitive Zellen das Neurotoxin erkannt haben und Antikörper gebildet wurden, werden die diesbezüglichen Gedächtniszellen über Jahre erhalten bleiben. Darum kommt es darauf an, die Patienten mit Präparaten höchster Aktivität in möglichst geringer Dosierung zu behandeln. Die Präparate sollten ferner keine weiteren Proteine bakterieller Herkunft enthalten, da diese als Immunadjuvantien wirken können. Solche Stoffe locken Makrophagen an, die sowohl die Immunadjuvantien als auch die Neurotoxine erkennen und den Lymphozyten präsentieren, welche daraufhin mit der Bildung von Immunglobulinen antworten. Folglich dürfen nur Produkte von höchster Reinheit, die keine Fremdproteine enthalten, für die Therapie Verwendung finden.

Ihara et al. (siehe Ihara et al. (2003), Biochim. Biophys Acta 1625 (1): 19-26) beschreibt die Sequenz des Gens für *Clostridium botulinum* Typ B Neurotoxin, die mit kindlichem Botulismus, der Expression der C-terminalen Hälfte der schweren Kette und deren Bindungsaktivität assoziiert ist. Ginalski et al. (siehe Ginalski et al. (2000), FEBS Lett. 482 (1-2):119-124) beschriebt dass die Struktur-basierte Sequenz-Gegenüberstellung für die beta-Kleeblatt-Unterdomäne der clostridialen Neurotoxin-Familie Informationen im Maßstab einzelner Reste über die putative Gangliosidbindugsstelle gewährt. Rummel et al. (siehe Rummel et al. (2004), Mol. Microbiol. 51 (3): 631-643) beschreibt dass die H_{CC}-Domäne der Botulinum Neurotoxine A und B eine einzige Gangliosidbindungsstelle aufweist, die Serotyp-spezifische Kohlenhydrat-Interaktion zeigt. Goodnough et al. (siehe Goodnough et al. (2002), FEBS Lett. 513 (2-3): 163-168) beschreibt die Entwicklung eines Übertragungsvehikels zum intrazellulären Transport von Botulinum Neurotoxin Antagonisten. Die WO 00/55208 offenbart ein chimäres Toxin, das die schwere Kette eines botulinen Neurotoxins und eine nicht-clostridiale Toxinkette umfasst.

Mit der vorliegenden Erfindung wird nun ein Transportprotein (Trapo) bereitgestellt, das die oben geschilderten Probleme der bisher bekannten Methoden überwinden kann.

Gemäß einem Aspekt umfasst die Erfindung dabei ein Transportprotein, erhältlich durch Modifizierung der schweren Kette des von *Clostridium botulinum* gebildeten Neurotoxins, wobei das Neurotoxin Botulinum Neurotoxin Typ A (BoNT/A) ist und wobei das Transportprotein an Nervenzellen mit höherer Affinität bindet als das native Neurotoxin, wobei die Bindungsaktivität im Synaptosomen-Assay bestimmt wird, und wobei mindestens eine Aminosäure in den Positionen 1117, 1252, 1253, 1270, 1278 bis 1279 Botulinum Neurotoxin Typ A Proteinsequenzen entfernt oder durch eine Aminosäure substituiert ist, die entweder natürlich vorkommt oder nicht natürlichen Ursprungs ist.

Gemäß einem weiteren Aspekt umfasst die Erfindung ferner ein Transportprotein, erhältlich durch Modifizierung der schweren Kette des *Clostridium botulinum* Neurotoxins Typ A (BoNT/A), wobei die Aminosäuren 1092 bis 1296 des *Clostridium botulinum* Neurotoxin Typ A, die die gangliosidbindende Domäne enthalten, ersetzt sind durch nachfolgende Sequenzen: *Clostridium botulinum* Neurotoxin Typ B Protein, Aminosäuren 1079 bis 1291, *Clostridium botulinum* Neurotoxin Typ C₁ Protein, Aminosäuren 1093 bis 1291, *Clostridium tetani* Neurotoxin Protein, Aminosäuren 1111 bis 1315.

Gemäß einem weiteren Aspekt umfasst die Erfindung außerdem ein Transportprotein, erhältlich durch Modifizierung der schweren Kette des *Clostridium botulinum* Neurotoxins Typ A (BoNT/A), wobei das vollständige H_{C}-Fragment von BoNT/A (Aminosäuren 867 bis 1296) durch das H_{C}-Fragment von BoNT/B (Aminosäuren 866 bis 1291) ersetzt ist.

"Bindung an Nervenzellen mit höherer Affinität als natives Neurotoxin". Das native Neurotoxin ist hierbei das native Neurotoxin von *C. botulinum* Typ A. Das rekombinant hergestellte Botulinus Neurotoxin aus *E. coli*, welches u. a. die zum nativen Botulinus Neurotoxin identische Aminosäuresequenz beinhaltet, verhält sich pharmakologisch identisch wie das native Botulinus Neurotoxin und wird rekombinantes Botulinus Neurotoxin Wildtyp genannt. Die hierbei erwähnten Nervenzellen sind cholinerge Motoneurone. Vorzugsweise bindet das Transportprotein spezifisch an Polysialoganglioside auf Nervenzellmembranoberflächen, wie z. B. GD1a, GD1b oder GT1b. Die Bindung wird vorzugsweise *in vitro* bestimmt. Besonders bevorzugt erfolgt die Bestimmung durch Verwendung eines Assays, der im Detail in den Beispielen ausgeführt ist.

Der Ausdruck "Modifizierung der schweren Kette des von *C. botulinum* gebildeten Neurotoxins". Die Aminosäure- und/oder Nuldeinsäuresequenz der schweren Kette (HC) des von *C. botulinum* gebildeten Neurotoxins sind allgemein aus öffentlich zugänglichen Datenbanken erhältlich, für jeden der bekannten Serotypen A bis G (siehe auch Tabelle 1). Modifizierung umfasst hierbei, dass wenigstens eine Aminosäure deletiert, addiert, in die Aminosäuresequenz insertiert ist, oder wenigstens eine Aminosäure des nativen Neurotoxins durch eine andere natürliche oder nicht natürlich vorkommende Aminosäure substituiert ist und/oder eine Aminosäure in der gegebenen Aminosäuresequenz posttranslational modifiziert ist. Posttranslationale Modifikationen umfassen hierbei Glykosylierungen, Acetylierungen, Acylierungen, Desaminierungen, Phosphorylierungen, Isoprenylierungen, Glykosylphosphatidylinositolierungen, und weitere dem Fachmann bekannte Modifikationen.

Die HC des von *C. botulinum* gebildeten Neurotoxins umfasst drei Subdomänen, nämlich die amino-terminale 50 kDa große Translokationsdomäne H_{N}, die sich anschließende 25 kDa H_{CN}-Domäne und die carboxyl-terminal gelegene 25 kDa H_{CC}-Domäne. Zusanrnmengefasst werden die H_{CN}- und H_{CC}-Domäne als H_{CC}-Fragment bezeichnet. Die entsprechenden Aminosäureabschnitte der jeweiligen Subdomänen sind für die einzelnen Serotypen und ihren Varianten aus Tabelle 1 ersichtlich.

Zur detaillierten Beschreibung von Hybridproteinen mit Domänen aus unterschiedlichen BoNT Serotypen wird im Nachfolgenden folgende Nomenldatur eingeführt. Der Ausdruck scAtAAB bezeichnet z. B. ein einkettiges Neurotoxin (sc), bestehend aus den vier Domänen LC, H_{N}, H_{CN} und H_{CC}, wobei jede Domäne entsprechend ihrer Abstammung mit dem Großbuchstaben des jeweiligen Serotyps gekennzeichnet ist Das heißt, dass in scAtAAB die LC, H_{N} und H_{CN} von BoNT/A abstammen, während die H_{CC}-Domäne von BoNT/A gegen die von BoNT/B ausgetauscht wurde. Der Kleinbuchstabe "t" symbolisiert eine eingefügte Thrombinerkennungssequenz zwischen LC und H_{N}.

**Tabelle 1: Datenbanknummern der Aminosäuresequenzen und Aufteilung der Subdomänen der sieben Botulinus Neurotoxine.**

| BoNT | Datenbanknr. der Protein-sequenz | Anzahl der Aminosäuren | HC | | |
|---|---|---|---|---|---|
| | | | H_{N} | H_{C} | |
| | | | | H_{CN} | H_{CC} |
| BoNT/A | AAA23262 | 1296 | 449-866 | 867-1091 | 1092-1296 |
| | AAM75961 | | | | |
| | AAQ06331 | | | | |
| | BTCLAB | | | | |
| | P10845 | 1296 | 449-866 | 867-1091 | 1092-1296 |
| | CAA36289 | 1296 | 449-866 | 867-1091 | 1092-1296 |
| | CAA51824 | 1296 | 449-866 | 867-1091 | 1092-1296 |
| | I40645 | | | | |
| | Q45894 | | | | |
| BoNT/B | AAL11499 | 1291 | 442-855 | 866-1078 | 1079-1291 |
| | AAL11498 | | | | |
| | CAA73968 | 1291 | 442-855 | 866-1078 | 1079-1291 |
| | AAK97132 | 1291 | 442-855 | 866-1078 | 1079-1291 |
| | A48940 | | | | |
| | AAA23211 | 1291 | 442-855 | 866-1078 | 1079-1291 |
| | P10844 | | | | |
| | BAC22064 | 1291 | 442-855 | 866-1078 | 1079-1291 |
| | CAA50482 | 1291 | 442-855 | | 1079-1291 |
| | 140631 | | | 866-1078 | |
| BoNT/C1 | A49777 | | | | |
| | BAA14235 | | | | |
| | BAB71749 | 1291 | 450-863 | 864-1092 | 1093-1291 |
| | CAA51313 | | | | |
| | S46431 | | | | |
| | P18640 | 1291 | 450-863 | 864-1092 | 1093-1291 |
| | BAA08418 | 1280 | 450-863 | 864-1083 | 1084-1280 |
| | BAA89713 | 1280 | 450-863 | 864-1083 | 1084-1280 |
| BoNT/D | CAA38175 | | | | |
| | P19321 | 1276 | 446-859 | 860-1079 | 1080-1276 |
| | S11455 | | | | |
| | AAB24244 | 1276 | 446-859 | 860-1079 | 1080-1276 |
| | BAA07477 | 1285 | 446-859 | 860-1088 | 1089-1285 |
| | S70582 | | | | |
| | BAA90661 | 1285 | 446-859 | 860-1088 | 1089-1285 |
| BoNT/E | BAB86845 | | | | |
| | CAA44558 | 1252 | 423-842 | 843-1066 | 1067-1252 |
| | S21178 | | | | |
| | CAA43999 | 1251 | 423-842 | 843-1066 | 1067-1251 |
| | Q00496 | | | | |
| | CAA43998 | | | | |
| | JH0256 | 1251 | 423-842 | 843-1066 | 1067-1251 |
| | P30995 | | | | |
| BoNT/F | 1904210A | 1274 | 440-860 | 861-1086 | 1087-1274 |
| | AAA23263 | | | | |
| | 140813 | | | | |
| | P30996 | | | | |
| | CAA73972 | 1280 | 440-861 | 862-1087 | 1088-1280 |
| | AAA23210 | 1278 | 440-861 | 862-1084 | 1085-1278 |
| | CAA57358 | | | | |
| | CAA48329 | 1268 | 432-853 | 854-1075 | 1076-1268 |
| | 533411 | | | | |
| BoNT/G | CAA52275 | | | | |
| | Q60393 | 1297 | 447-860 | 861-1086 | 1087-1297 |
| | S39791 | | | | |

Die vorliegende Erfindung bezieht sich insbesondere auf ein Transportprotein, welches durch Modifizierung der HC des von *Clostridium Botulinum* gebildeten Neurotoxins erhalten wird, wobei das besagte Protein mit höherer Affinität als das native Neurotoxin spezifisch an Nervenzellen bindet und von diesen Zellen durch Endozytose aufgenommen wird.

Das in der vorliegenden Erfindung bereitgestellte Transportprotein weist eine erhöhte spezifische Affinität seiner gangliosidbindenden Domäne zu komplexen Polysialogangliosiden auf. Die Erhöhung der Affinität wird vorzugsweise durch Substitution oder Deletion von Aminosäuren erzielt.

Gemäß einer bevorzugten Ausführungsform weist das Transportprotein eine wenigstens 15 % höhere Affinität als das native Neurotoxin auf. Vorzugsweise weist das Transportprotein eine wenigstens 50 % höhere, besonders bevorzugt wenigstens 80 % höhere, und insbesondere eine wenigstens 90 % höhere Affinität als das native Neurotoxin auf.

Erfindungsgemäβ erfolgt die Modifizierung der HC im Bereich des H_{C}-Fragmentes des gegebenen Neurotoxins. Sofern die Modifikation eine Substitution, Deletion, Insertion oder Addition umfasst, kann diese beispielsweise durch zielgerichtete Mutagenese durchgeführt werden, Verfahren hierzu sind dem Fachmann bekannt. Die in dem nativen Neurotoxin vorhandenen Aminosäuren werden hierbei entweder durch natürlich vorkommende oder nicht natürlich vorkommende Aminosäuren verändert. Grundsätzlich werden Aminosäuren in unterschiedliche physikochemische Gruppen eingeteilt. Zu den negativ geladenen Aminosäuren gehören Aspartat und Glutamat. Zu den positiv geladenen Aminosäuren gehören Histidin, Arginin und Lysin. Zu den polaren Aminosäuren gehören Asparagin, Glutamin, Serin, Threonin, Cystein und Tyrosin. Zu den unpolaren Aminosäuren gehören Glycin, Alanin, Valin, Leucin, Isoleucin, Methionin, Prolin, Phenylalanin und Tryptophan. Aromatische Seitengruppen finden sich bei den Aminosäuren Histidin, Phenylalanin, Tyrosin und Tryptophan. Generell ist bevorzugt, dass eine Aminosäure durch eine andere Aminosäure ausgetauscht wird, die zu einer anderen physikochemischen Gruppe gehört.

Erfindungsgemäβ ist das Transportprotein von der Proteinsequenz des *Clostridium botulinum* Neurotoxins vom Typ A (Datenbanknr. AAA23262 und CAA51824) abgeleitet.

Die orliegende Erfindung bezieht sich auf ein Transportprotein, bei dem zumindest eine Aminosäure in den Positionen 1117, 1252, 1253, 1270 und 1278 bis 1279 der Proteinsequenz des *Clostridium botulinum* Neurotoxins vom Typ A (Datenbanknr. AAA23262 und CAA51824) entweder entfernt oder durch eine natürlich vorkommende oder nicht natürlich vorkommende Aminosäure ersetzt wurde.

Eine weitere Ausführungsform der vorliegenden Erfindung bezieht sich auf ein Transportprotein, bei dem Aminosäuren in den Positionen 1092 bis 1296 der Proteinsequenz des *Clostridium botulinum* Neurotoxins vom Typ A (Datenbanknr. AAA23262 und CAA51824) - ein Bereich, der die gangliosidbindende Domäne umfasst - ersetzt sind durch die Sequenz von *Clostridium botulinum* Neurotoxin Typ B Protein (Datenbanknr. AAA23211), Aminosäuren 1079 bis 1291, *Clostridium botulinum* Neurotoxin Typ C₁ Protein (Datenbanknr. CAA51313), Aminosäuren 1093 bis 1291,

Weitere für den Austausch geeignete H_{CC}-Domänen mit Aminosäurepositionen sind Tabelle 1 zu entnehmen.

Eine weitere Ausführungsform der vorliegenden Erfindung bezieht sich auf eine Zusammensetzung, die ein erfindungsgemäßes Transportprotein und wenigstens ein intervenierendes Molekül (X) enthält. Das intervenierende Molekül kann ein kleines organisches Molekül, ein Peptid oder ein Protein sein; vorzugsweise kovalent gebunden, z.B. durch eine Peptid-, Ester-, Ether-, Sulfid-, Disulfid- oder Kohlenstoff-Kohlenstoff-Bindung.

Das intervenierende Molekül umfasst zusätzlich alle bekannten therapeutisch wirksamen Stoffe. Bevorzugt sind hierbei Zytostatika, Antibiotika, Virustatika, aber auch Immunglobuline.

Um die erhöhte Affinität des Trapo besser auszunutzen, wurde es amino-terminal über eine Aminosäuresequenz, die spezifisch von der Protease Thrombin erkannt und gespalten wird, an eine LC von BoNT/A gebunden, wodurch ein bestimmtes TrapoX entstand. Die Wirkstärke des besagten TrapoX war gegenüber nativem BoNT/A um einen Faktor von besonders bevorzugt mindestens 3 erhöht. Dieses neue Produkt, das frei von Fremdproteinen ist, wird die Stimulation des Immunsystems aufgrund der größeren Reinheit des Materials und der niedrigeren Dosierung dramatisch vermindern.

Eine weitere Ausführungsform der vorliegenden Erfindung bezieht sich auf ein Transportprotein, in dem das Protein eine Protease ist, die ein oder mehrere Proteine des Freisetzungsapparates von Neurotransmittern spaltet, wobei die Protease aus der Gruppe der Neurotoxine ausgewählt ist, die aus den LC der *Clostridium botulinum* Neurotoxine, insbesondere des Typs A, B, C₁, D, E, F und G oder einem proteolytisch aktivem Fragment der LC eines *Clostridium botulinum* Neurotoxins, insbesondere eines Neurotoxins des Serotyps A, B, C₁, D, E, F und G besteht, wobei das Fragment wenigstens 0,01 % der proteolytischen Aktivität der nativen Protease, vorzugsweise wenigstens 5 %, besonders bevorzugt wenigstens 50 %, insbesondere wenigstens 90 % aufweist. Vorzugsweise ist das Transportprotein und die Protease vom gleichen *C. Botulinum* Neurotoxin Serotyp abgeleitet, insbesondere bevorzugt ist die H_{N}-Domäne des Transportproteins und die Protease vom *C. botulinum* Neurotoxin Serotyp A abgeleitet. Die Sequenzen der Proteasen sind allgemein aus Datenbanken zugänglich und die Datenbanknummern sind aus Tabelle 1 ersichtlich. Die proteolytische Aktivität der Proteasen wird anhand einer Substratspaltungskinetik bestimmt (siehe Binz et al. (2002), Biochemistry 41(6), 1717-23).

Die LCs sind dadurch charakterisiert, dass sie die Sequenz His-Glu-Leu-Xaa-His-(Xaa)₃₃₋₃₅-Glu-(Xaa)₈₄₋₉₀-Glu-(Xaa)₁₁-Arg-Xaa-Xaa-Tyr enthalten, wobei Xaa irgendeine Aminosäure sein kann. Das Transportprotein der vorliegenden Erfindung ist dadurch charakterisiert, dass das. Protein und die Protease aus den vorherigen Gruppen von Proteinen bzw. Proteasen abstammen.

Gemäß einer weiteren Ausführungsform der vorliegenden Erfindung wird ein Verfahren zur Herstellung des Transportproteins bereitgestellt. In einem ersten Schritt wird hierbei eine das Transportprotein kodierende Nukleinsäure bereitgestellt. Die kodierende Nukleinsäure kann hierbei RNA, DNA oder Gemische davon darstellen. Die Nukleinsäure kann ferner im Hinblick auf ihre Nuldeaseresistenz modifiziert sein wie z. B. durch Einfügung von Phosphorthioat-Bindungen. Die Nukleinsäure kann aus einer Ausgangs-Nuldeinsäure hergestellt werden, wobei die Ausgangs-Nukleinsäure z. B. durch Klonierung aus genomischen oder cDNA-Banken zugänglich ist. Weiterhin kann die Nukleinsäure direkt durch Festphasensynthese hergestellt werden. Geeignete Verfahren sind dem Fachmann bekannte. Sofern von einer Ausgangs-Nukleinsäure ausgegangen wird, kann z. B. durch ortsgerichtete Mutagenese eine zielgerichtete Veränderung durchgeführt werden, die auf Aminosäure-Ebene zu wenigstens einer Addition, Insertion, Deletion und/oder Substitution führt. Die Nukleinsäure wird sodann in operativer Weise mit einem geeigneten Promotor verbunden. Geeignete Promotoren für die Expression in bekannten Expressionssystemen sind dem Fachmann bekannt. Die Wahl des Promotors hängt hierbei vom zur Expression verwendeten Expressionssystem ab. Generell sind konstitutive Promotoren bevorzugt, jedoch sind auch induzierbare Promotoren verwendbar. Das so hergestellte Konstrukt umfasst wenigstens einen Teil eines Vektors, insbesondere regulatorische Elemente, wobei der Vektor beispielsweise ausgewählt ist aus λ-Derivaten, Adenoviren, Baculoviren, Vacciniaviren, SV40-Viren und Retroviren. Der Vektor ist vorzugsweise zur Expression der Nukleinsäure in einer gegebenen Wirtszelle fähig. Ferner stellt die Erfindung Wirtszellen bereit, die den Vektor enthalten und die zur Expression des Vektors geeignet sind. Im Stand der Technik sind zahlreiche prokaryontische und eukaryontische Expressionssysteme bekannt, wobei die Wirtszellen beispielsweise ausgewählt sind aus prokaryontischen Zellen wie *E. coli* oder *B. megaterium*, aus eukaryontischen Zellen wie *S. cerevisiae* und *P. pastoris*. Zwar können auch höhere eukaryontischen Zellen verwendet werden wie Insektenzellen oder Säugerzellen, jedoch sind Wirtszellen bevorzugt, die ebenso wie *C. Botulinum* über keinen Glykosylierungs-Apparat verfügen.

Sofern eine erfindungsgemäße Zusammensetzung bereitgestellt wird, die neben dem Transportprotein noch wenigstens ein intervenierendes Molekül enthält, und dieses intervenierende Molekül ein Peptid oder Protein entweder mit einem carboxyl-telminalen Cystein oder einer Mercapto-Gruppe funktionalisiert ist, so kann in analoger Weise wie zuvor beschrieben das Peptid bzw. das Protein rekombinant hergestellt werden, beispielsweise unter Verwendung von binären Vektoren oder durch unterschiedliche Wirtszellen. Sofern dieselbe Wirtszelle für die Expression sowohl des Transportproteins als auch des Peptids oder Proteins verwendet wird, wird vorzugsweise eine intermolekulare Disulfid-Bindung *in situ* gebildet. Zur effizienteren Produktion in derselben Wirtszelle kann die das Peptid oder Protein kodierende Nukleinsäure auch mit der des Transportproteins im gleichen Leserahmen translatiert werden, so dass ein einkettiges Polypeptid produziert wird. Hierbei bildet sich dann vorzugsweise eine intramolekulare Disulfid-Bindung *in situ*. Zur einfachen Hydrolyse der ebenfalls vorhanden peptidischen Verknüpfung zwischen Transportprotein und Peptid bzw. Protein wird am Amino-Terminus des Transportproteins eine Aminosäuresequenz, die entweder spezifisch von der Protease Thrombin oder einer spezifischen Endoprotease der Wirtszelle erkannt und gespalten wird, eingefügt.

Sofern dieses nicht möglich ist, kann nach separater Aufreinigung des Transportproteins und des Proteins nachfolgend durch dem Fachmann bekannte Oxidationsverfahren eine entsprechende intermolekulare Disulfid-Bindung herbeigeführt werden. Das Peptid bzw. Protein kann auch direkt durch Synthese oder Fragmentkondensation erhalten werden. Entsprechende Verfahren sind dem Fachmann bekannt.

Das Transportprotein und das Peptid bzw. Protein werden nachfolgend aufgereinigt. Hierbei kommen dem Fachmann bekannte Verfahren zum Einsatz, wie z. B. Chromatographie-Verfahren oder Elektrophorese.

Eine weitere Ausführungsform der vorliegenden Erfindung bezieht sich auf die pharmazeutische Zusammensetzung, die das Transportprotein und optional einen pharmazeutisch akzeptablen Träger, ein Verdünnungsmittel und/oder Additiv einschließt und zur Behandlung der folgenden Störungen und Erkrankungen geeignet ist: Hemifacialspasmus, spasmodischer Torticollis, Spastizitäten, Dystonien, Migräne, Schmerzen, Erkrankungen der Hals- und Lendenwirbelsäule, Strabismus, Hypersalivation und depressive Erkrankungen.

Die pharmazeutische Zusammensetzung ist zur oralen, intravenösen, subkutanen, intramuskulären und topischen Verabreichung geeignet. Die intramuskuläre Verabreichung ist hierbei bevorzugt. Eine Dosiseinheit der pharmazeutischen Zusammensetzung enthält ungefähr 0,1 pg bis 1 mg Transportprotein und/oder die erfindungsgemäße Zusammensetzung.

Eine weitere Ausführungsform der vorliegenden Erfindung schließt eine kosmetische Zusammensetzung ein, die aus dem Transportprotein und einem pharmazeutisch Träger, Verdünnungsmittel und/oder Additiv besteht, geeignet für die Behandlung von Hyperhydrose und stark ausgeprägten Gesichtsfalten. Die kosmetische Zusammensetzung ist zur oralen, intravenösen, subkutanen, intramuskulären und topischen Verabreichung geeignet. Die intramuskuläre Verabreichung ist hierbei bevorzugt. Eine Dosiseinheit der kosmetischen Zusammensetzung enthält ungefähr 0,1 pg bis 1 mg Transportprotein und/oder die erfindungsgemäße Zusammensetzung. Die kosmetische Zusammensetzung ist geeignet für die Behandlung von Hyperhydrose und Gesichtsfalten.

Das in der vorliegenden Erfindung beschriebene Transportprotein kann von einer geeigneten Wirtszelle, wie z.B. *Escherichia coli, Saccharomyces cerevisiae, Pichia pastoris* oder *Bacillus megaterium* hergestellt werden, die einen rekombinanten Expressionsvektor vervielfältigt wobei der Vektor ein Transportprotein codiert.

Die vorliegende Erfindung wird auch durch die beigefügten Abbildungen verdeutlicht, wobei:
Figur 1 zeigt, dass die Affinität des mutierten H_{C}-Fragmentes von BoNT/A zu Synaptosomemnembranpräparationen aus dem Rattenhirn dreifach höher ist als die des H_{C}-Fragments des Wildtyps von BoNT/A.
Figur 2 zeigt die Bindung verschiedener BoNT/A H_{C}-Fragment Muteine an Rattenhimsynaptosomen, wobei die Affinität des BoNT/A H_{C}-Fragment Wildtyps als Standard auf 100% gesetzt wurde. Im ersten Block sind die Affinitäten der BoNT/A-Muteine dargestellt, die Mutationen der Aminosäuren Y1117 zeigen, die zu einer Erhöhungen führen. Im zweiten Block sind weitere BoNT/A-Muteine dargestellt. Im dritten Block sind die Affinitäten von BoNT/A-Muteinen gezeigt, die Doppelmutationen aufweisen, welche die Bindung an Nervenzellmembranen (Synaptosomen) verstärken.
Figur 3 zeigt die erhöhte Neurotoxizität des Y1117A-Muteins von BoNT/A im Vergleich zum BoNT/A-Wildtyp am isolierten Nervus phrenicus - Zwerchfellmuslcel-Präparat der Maus.
Figur 4 zeigt die Bindung der vier BoNT/A H_{c}-Fragmenthybride H_{c}AB, H_{c}AC, H_{c}AE und H_{c}AT (T = Tetanus Neurotoxin) an Nervenzellenmembranen (Synaptosomen), wobei der BoNT/A H_{c}-Fragment Wildtyp als Standard auf 100% gesetzt ist.
Figur 5 zeigt die erhöhte Neurotoxizität der Gesamttoxinhybride bestehend aus BoNT/A und entweder dem H_{c}-Fragment oder der H_{cc}-Domäne von BoNT/B im Vergleich zu BoNT/A-Wildtyp am isolierten Nervus phrenicus - Zwerchfellmuskel-Präparat der Maus.

Im Detail beinhaltet die vorliegende Erfindung ein Transportprotein (Trapo), das durch Modifizierung der HC des von *Clostridium botulinum* produzierten Neurotoxins Typ A entsteht, vorzugsweise spezifisch an Neurone bindet, durch rezeptorvermittelte Endozytose intrazellulär aufgenommen wird und aus dem sauren endosomalen Kompartiment in das Zytosol von Neuronen transloziert wird. Dieses Protein wird als Transporter benutzt, um daran gebundene Proteasen und andere Substanzen, die physiologisch die Plasmamembran nicht durchdringen und in das Zytosol von Nervenzellen gelangen können, in die Zellen einzuschleusen. Die Substrate der Proteasen sind intrazellulär lokalisierte Proteine und Peptide, die an der Transmitterfreisetzung beteiligt sind. Nach Spaltung der Substrate sind die spezifischen Funktionen der Neurone blockiert, wobei die Zellen selbst nicht geschädigt sind. Eine dieser Funktionen ist die Exozytose, die die Neurotransmitterfreisetzung bewerkstelligt. Ist die Freisetzung von Transmittern gehemmt, ist die Übermittlung von Signalen von Zelle zu Zelle blockiert. Beispielsweise werden gestreifte Muskeln gelähmt, wenn die Freisetzung von Acetylcholin an der neuromuskulären Kontaktstelle gehemmt ist. Diese Wirkung kann therapeutisch genutzt werden, wenn TrapoX an Nervenendigungen spastischer oder dystoner Muskeln appliziert wird. Andere aktive Substanzen sind beispielsweise Wirkstoffe mit antiviraler Wirkung. Mit Trapo konjugiert, könnten sie für die Behandlung viraler Infektionen des Nervensystems von Nutzen sein. Die vorliegende Erfindung bezieht sich auch auf den Gebrauch eines Transportproteins zur Hemmung der Freisetzung von Neurotransmittern.

Wenn Patienten mit den nativen Formen von BoNT/A und B behandelt werden, verursacht die Injektion dieser nicht humanen Proteine trotz der niedrigen Dosis die Bildung von Antikörpern, so dass die Therapie abgebrochen werden muss, um einen anaphylaktischen Schock zu vermeiden. Indem man eine Substanz desselben Wirkmechanismus mit einer höheren Transporteffizienz der enzymatischen Aktivität appliziert, kann die Dosis drastisch gesenkt werden, und es wird nicht zur Bildung von Antikörpern kommen. Diese Eigenschaften kommen dem hier beschriebenen Transportprotein zu.

Auch wenn Beispiele für die Applikation angegeben werden, so werden im Allgemeinen der geeignete Applikationsmodus und die Dosierung von dem behandelnden Arzt individuell bestimmt. Solche Entscheidungen werden routinemäßig von jedem im Fachgebiet bewanderten Arzt getroffen. So kann z.B. der Applikationsmodus und die Dosierung des Neurotoxins gemäß der hier dargelegten Erfindung auf der Basis von Kriterien wie der Löslichkeit des gewählten Neurotoxins oder der Intensität der zu behandelnden Schmerzen gewählt werden.

Gegenwärtig beträgt das Behandlungsintervall für natives BoNT/A und B durchschnittlich drei bis vier Monate. Die Verlängerung dieses Intervalls würde das Risiko der Antikörperbildung vermindern und eine längere Dauer der Behandlung mit BoNT ermöglichen. Die Zunahme von LC im Zytosol würde deren Abbau zeitlich strecken und damit auch die Wirkdauer verlängern. Das hier beschriebene Transportprotein besitzt eine höhere Affinität und Aufnahmerate als die native HC-A.

Das folgende Beispiel dient lediglich dem Zwecke der Verdeutlichung und sollte nicht als begrenzend angesehen werden.

### Beispiele

### Rekombinante Expression des gentechnisch hergestellten TrapoX in E. coli

Die DNA-Sequenz der HC von BoNT/A wurde an chromosomaler DNA von *Clostridium botulinum* (Datenbanknr. AAA23262) mittels PCR amplifiziert. Hierbei wurde das Kodon für die Aminosäure Tyrosin 1117 mit Hilfe von spezifischen Oligonukleotiden durch ein Basentriplett ersetzt, das den Aminosäurerest von Alanin kodiert. Weiterhin wurde das 5'-Ende des Gens mit einer DNA-Sequenz ergänzt, welche die Aminosäuren der Erkennungssequenz von Thrombin kodiert. Diese DNA-Sequenz wurde in einen bakteriellen Expressionsvektor eingefügt. Dabei wurde das eingefügte Gen für Trapo am 3'-Ende mit einem Oligonuldeotid fusioniert, das ein carboxy-terminales Affinitätspeptid wie z.B. den StrepTag, 6xHN-Tag oder His₆-Tag kodiert. Der auf diese Weise erzeugte Expressionsvektor pAR-Trapo ist die Ausgangsbasis für das Anfügen von Trägermolekülen, wie den LC der BoNT.

Die DNA-Sequenz der LC von BoNT/A wurde an chromosomaler DNA-Sequenz von *Clostridium botulinum* (Datenbanknr. AAA23262) mit der PCR-Methode amplifiziert und in den Expressionsvektor pAR-Trapo stromaufwärts der kodierten Thrombinerkennungssequenz eingefügt. Der so entstandene Expressionsvektor pAR-TrapoX wurde in einen *E. coli* K12 Stamm transformiert und die Expression des Proteins TrapoX unter den Bedingungen der Biologischen Sicherheitsstufe 2 und unter Einhaltung der für das Projekt erlassenen Vorschriften der Bezirksregierung Hannover, Az 501g.40654/3/57/3, induziert. Das überexprimierte TrapoX wurde als einkettiges Protein mit einem Molekulargewicht von 150 kDa affinitätschromatographisch nach Anleitung des Herstellers isoliert. Nachfolgend wurde das Protein mit an Sepharose konjugiertem Thrombin hydrolysiert, wobei ein reines Protein entstand, dessen zwei Ketten durch eine Disulfid-Brücke miteinander verbunden blieben.

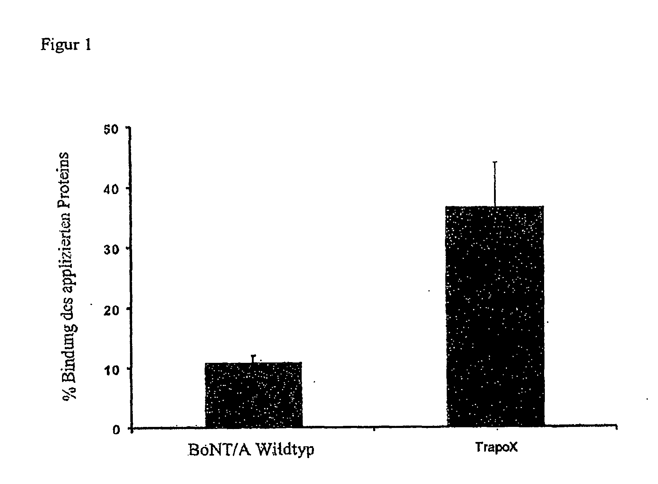

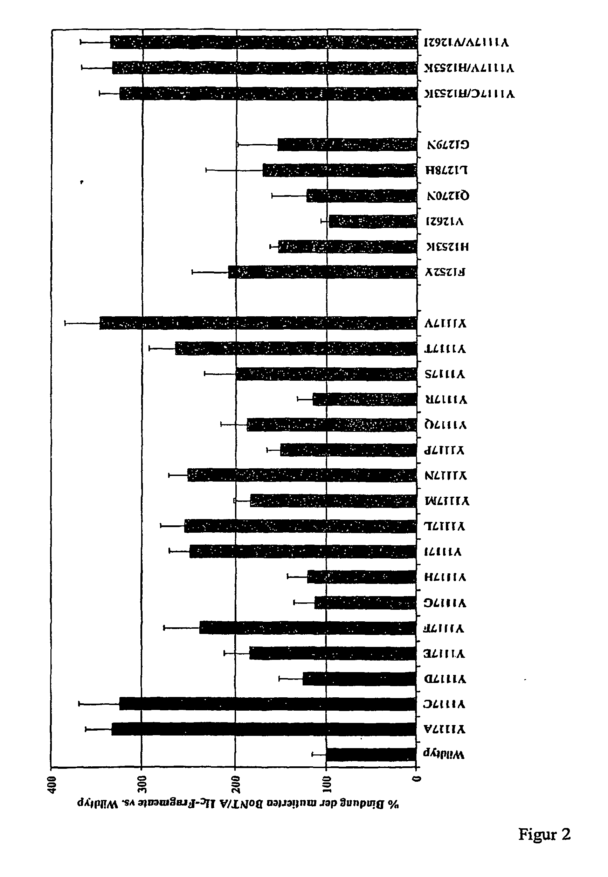

Dieses Protein wies gegenüber dem Wildtyp von BoNT/A eine um 300% erhöhte Affinität zu isoliertem, an Microtiterplatten immobilisiertem Gangliosid GT1b und zu Synaptosomenmembranpräparationen aus Rattenhim auf (Figur 1). Die katalytische Aktivität der LC-A war nicht verändert, wie sich an der *in vitro* Spaltung von rekombinantern SNAP-25 zeigte. Die Potenz des TrapoX hinsichtlich der Hemmung der Neurotransmitterfreisetzung in funktionalen Synaptosomen aus Rattenhim war, verglichen mit dem aus *Clostridium botulinum* gewonnenem nativem BoNT/A, um 300% gesteigert. An Nerv-Muskel-Präparaten der Maus (HDA) war die Potenz des TrapoX gleichfalls um 300% gegenüber dem nativen BoNT/A erhöht (Figur 2).

### Messung der Bindung an Rattenhirnsynaptosomen und der Neurotoxizität im HDA verschiedener BoNT/A-Muteine

Die Bindung radioaktiv markierter H_{c}-Fragmente an Rattensynaptosomen wurde gemessen wie angegeben in Rummel et al., J. Mol. Biol. 326 (2003), 835-847. Die Neurotoxizität der BoNT/A-Muteine wurde bestimmt wie beschrieben von Habermann et al., Naunyn Schmiedeberg's Arch. Pharmacol. 311 (1980), 33-40.

Der Vergleich der Bindung der verschiedenen BoNT/A-Muteine im Vergleich zum Wildtyp ist in nachfolgender Tabelle gezeigt:

**Tabelle zu Figur 2**

| **Mutation** | **% vs. Wildtyp** | **Standardabweichung** |
|---|---|---|
| Wildtyp | 100,0 | *15,00* |
| Y1117A | 332,3 | *29, 00* |
| Y1117C | 324,2 | *44,75* |
| Y1117D | 124,4 | *26,94* |
| Y1117E | 183,3 | *27,95* |
| Y1117F | 235,9 | *38,41* |
| Y1117G | 112,8 | *21,34* |
| Y1117H | 120,0 | *22,29* |
| Y1117I | 248,1 | *21,95* |
| Y1117L | 253,6 | *25,65* |
| Y1117M | 182,8 | *18,41* |
| Y1117N | 250,3 | *20,13* |
| Y1117P | 150,3 | *14,98* |
| Y1117Q | 187,3 | *28,19* |
| Y1117R | 115,4 | *16,80* |
| Y1117S | 199,2 | *32,65* |
| Y1117T | 264,1 | *28,55* |
| Y1117V | 346,9 | *37,61* |
| | | |
| F1252Y | 208,0 | *38,36* |
| H1253K | 153,0 | *9,24* |
| V1262I | 97,8 | *9,38* |
| Q1270N | 122,3 | *37,81* |
| L1278H | 170,0 | *61,59* |
| G1279N | 153,6 | *44,54* |
| | | |
| Y1117C/H1253K | 324,8 | *22,72* |
| Y1117V/H1253K | 332,9 | *33,48* |

Die Mutation einzelner bestimmter Aminosäuren innerhalb der Gangliosidbindungstasche von BoNT/A führte zu einer Erhöhung der Bindung an Nervenzellen. Vorzugsweise wird an Position 1117 Tyrosin durch Alanin, Cystein oder Valin ersetzt. Insbesondere führt die Ersetzung des Tyrosinrestes an Position 1117 durch Alanin zu einer Erhöhung der Affinität auf ca. 330%.

Weitere Mutationen einzelner Aminosäuren aus der Gangliosidbindungstasche an Position 1252 und 1253 führten ebenfalls zu einer Erhöhung der Bindung. Insbesondere die Mutation von F1252 in Tyrosin und H1253 in Lysin führte zu einer Steigerung der Affinität um 110% bzw. 50%.

Weiterhin sind Erhöhungen der Bindung an Nervenzellen zu erwarten bei Mutationen an den Position 1202, 1262, 1270, 1278 und 1279.

Ferner wurden ebenfalls Muteine von BoNT/A mit Doppelmutationen getestet, wobei insbesondere die Muteine Y1117C/H1253K und Y1117V/H1253K zu einer Erhöhung der Bindung an Synaptosomen führten (vgl. Figur 2).

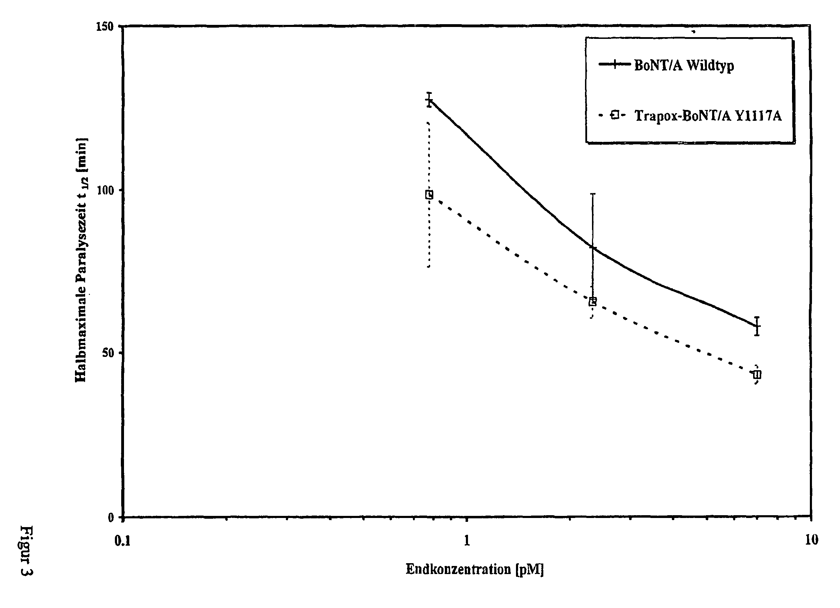

Ferner wurde bestimmt, dass die Erhöhung der Bindung insbesondere des Muteins Y1117A von BoNT/A zu einer Erhöhung der Neurotoxizität im N. phrenicus - Neutrotoxizitätsassay (HDA-Assay) führten (Figur 3).

### Bestimmung der Bindung und Neurotoxizität von BoNT/A H_{CC} Hybriden

Die Bestimmung der Bindung und der Neurotoxizität erfolgten wie vorstehend beschrieben.

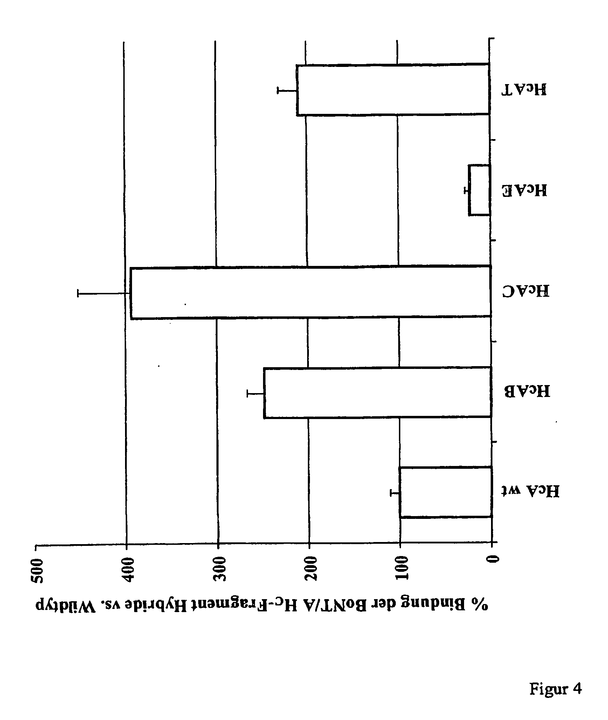

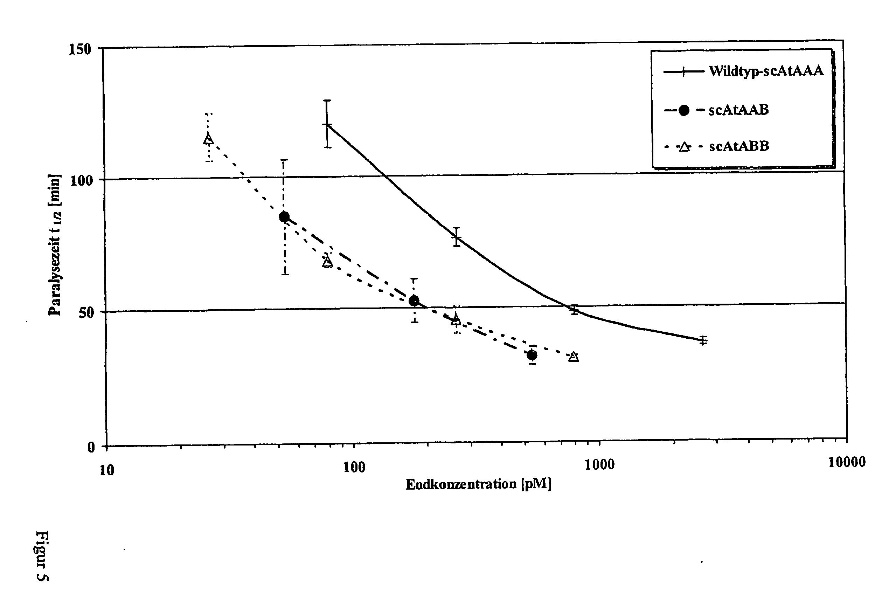

Die Ergebnisse sind in nachstehender Tabelle und ferner in den Figuren 4 und 5 dargestellt.

**Tabelle zur Figur 4**

| **Mutation** | **% vs. Wildtyp** | **Standardabweichung** |
|---|---|---|
| HcA wt | 100,0 | *10,4* |
| HcAB | 249,2 | *19,1* |
| HcAC | 393,4 | *57,9* |
| HcAE | 22,0 | *5,3* |
| HcAT | 210,2 | *22,5* |

Der Austausch der H_{cc}-Domäne von BoNT/A gegen die anderer Serotypen insbesondere *C. botulinum* Neurotoxin *B* und *C. botulinum* Neurotoxin C führte zu einer Erhöhung der Bindung an Nervenzellen. Weiterhin wurde beobachtet, dass der Austausch der H_{CC}-Domäne von H_{C}-Fragment von BoNT/A gegen die entsprechende Domäne von Tetanus Neurotoxin ebenfalls zu einer Erhöhung der Affinität an Nervenzellen führte. Die Affinitätsveränderungen gelten auch für den Austausch der H_{CC}-Domäne im gesamten BoNT/A. Figur 5 zeigt hierbei, dass beim Hybrid scAtAAB die Affinitätssteigerung sich in gleichem Maße auf eine Erhöhung der Neurotoxizität auswirkt. Wird anstelle der H_{CC}-Domäne das gesamte H_{C}-Fragment scAtAAB ausgetauscht, so werden entsprechende Ergebnisse beobachtet. Insbesondere wurde beobachtet, dass beim Austausch der H_{CC}-Domäne oder des H_{C}-Fragmentes von BoNT/A mit dem von BoNT/B eine Verbesserung der Neurotoxizität um ca. 350% beobachtet wurde.

### Bestimmung der Bindung der BoNT-Muteine an das Gangliosid GT1b

Gangliosid GT1b [NAcNeuα3Galβ3NAcGal β4(NAcNeuα8NAcNeuα3)Galβ4Glcβ] (Sigma-Aldrich) wird in Methanol gelöst und auf hochaffine 96-Napf Polystyrol-Mikrotiterplatten (Corning; 1µg GT1b in 100 µl /Napf) oder im Fall von Kompetitionassays mit ¹²⁵I-BoNTs auf hochaffine C8 Einzelbruchstreifenplatten (Greiner Bio-ohne; 0,1µg GT1b in 100µl / Napf) aufgebracht. Das Lösungsmittel wird bei Raumtemperatur verdampft und die Näpfe wurden dreimal mit Bindungspuffer (10mM Tris-HCl, 10mM Na₂HPO₄, 0,5% BSA, pH7.2) gewaschen. Die unspezifischen Bindungsstellen werden so dann durch Inkubieren über zwei Stunden in PBS/Tween [140 mM NaCl, 7 mM KCl, 10 mM Na₂HPO₄, 1,8 mM KH₂PO₄, 0,05% (V/V) Tween 20, pH 7,2] ergänzt mit 3% (w/v) BSA blockiert. Die Bindungsassays werden in Bindungspuffer (100µl / Napf) über 2 Stunden Raumtemperatur mit entweder ansteigenden Mengen von Wildtyp oder bestimmten Mengen der Mutanten durchgeführt. Ungebundenes Protein wird durch 3 Waschschritte entfernt, jeweils mit 250µl PBS/Tween Puffer. Gebundene H_{C}-Fragmente werden durch Inkubation mit Strep Tactin konjungiert mit alkalischer Phosphatase (ST-AP, IBA GmbH) nachgewiesen in Bindungspuffer über 2 Stunden bei Raumtemperatur gemäß Herstelleranleitung. p-Nitrophenylphosphat (1 mg/ml in 100 mM Glycin, 1 mM MgCl₂, 1 mM ZnCl₂, pH 10,4), das letztendlich als Substrat für die alkalische Phosphatase dient. Die Desphorphorylierungsreaktion wird durch Hinzufügen von 3 M NaOH Lösung gestoppt und die Extinktion bei 405 nm unter Verwendung eines Spectra Count Mikroplattenauslesegerätes (Packard) gemessen. Die Kompetitions-Assays werden über 2 Stunden bei Raumtemperatur in 100µl Bindungspuffer mit 700000cpm/Napf [125I]-BoNT, unterschiedlichen Mengen nativen BoNT oder rekombinantern H_{C}-Fragment durchgeführt. Nach Inkubation und Entfernung der Überstände werden die Näpfe dreimal mit PBS/Tweenpuffer gewaschen, getrocknet und getrennt. Die Mengen von gebundenem radioaktiv markierten BoNT werden dann in einem automatischen γ-Zähler bestimmt (Wallac 1480 Wizard 3).

## Patentansprüche

1. Ein Transportprotein, erhältlich durch Modifizierung der schweren Kette des von *Clostridium botulinum* gebildeten Neurotoxins, wobei das Neurotoxin Botulinum Neurotoxin Typ A (BoNT/A) ist und wobei das Transportprotein an Nervenzellen mit höherer Affinität bindet als das native Neurotoxin, wobei die Bindungsaktivität im Synaptosomen-Assay bestimmt wird, und wobei mindestens eine Aminosäure in den Positionen 1117, 1252, 1253, 1270, 1278 bis 1279 Botulinum Neurotoxin Typ A Proteinsequenzen entfernt oder durch eine Aminosäure substituiert ist, die entweder natürlich vorkommt oder nicht natürlichen Ursprungs ist.

2. Ein Transportprotein, erhältlich durch Modifizierung der schweren Kette des *Clostridium botulinum* Neurotoxins Typ A (BoNT/A), wobei das Transportprotein an Nervenzellen mit höherer Affinität bindet als das native Neurotoxin, wobei die Bindungsaktivität im Synaptosomen-Assay bestimmt wird, und wobei die Aminosäuren 1092 bis 1296 des *Clostridium botulinum* Neurotoxin Typ A, die die gangliosidbindende Domäne enthalten, ersetzt sind durch nachfolgende Sequenzen:
*Clostridium botulinum* Neurotoxin Typ B Protein, Aminosäuren 1079 bis 1291, oder
*Clostridium botulinum* Neurotoxin Typ C₁ Protein, Aminosäuren 1093 bis 1291, oder
H_{CC}-Domäne des *Clostridium tetani* Neurotoxin Proteins-.

3. Ein Transportprotein, erhältlich durch Modifizierung der schweren Kette des *Clostridium botulinum* Neurotoxins Typ A (BoNT/A), wobei das Transportprotein an Nervenzellen mit höherer Affinität bindet als das native Neurotoxin, wobei die Bindungsaktivität im Synaptosomen-Assay bestimmt wird, und wobei das vollständige H_{C}-Fragment von BoNT/A (Aminosäuren 867 bis 1296) durch das H_{C}-Fragment von BoNT/B (Aminosäuren 866 bis 1291) ersetzt ist.

4. Das Transportprotein nach Anspruch 1, 2 oder 3 wobei das Transportprotein spezifisch an Nervenzellen bindet und durch Endozytose von den Zellen aufgenommen wird.

5. Das Transportprotein nach Anspruch 1, 2 oder 3 wobei das Transportprotein spezifisch an komplexe Ganglioside cholinerger Motoneurone bindet, die in der Plasmamembran lokalisiert sind, vorzugsweise an GT1b.

6. Das Transportprotein nach einem der Ansprüche 1 bis 5, wobei das Transportprotein eine wenigstens 15% höhere Affinität als das native Neurotoxin aufweist, vorzugsweise wenigstens 50%, besonders bevorzugt wenigstens 80%, insbesondere wenigstens 90%.

7. Das Transportprotein nach Anspruch 1, wobei die Aminosäure in Position 1117 der Botulinum Neurotoxin Typ A Proteinsequenzen entfernt oder durch einer Aminosäure substituiert ist, die entweder natürlich vorkommt oder nicht natürlichen Ursprungs ist.

8. Das Transportprotein nach Anspruch 7, wobei die substituierte Aminosäure entweder Alanin, Cystein, Glutamat, Phenylalanin, Isoleucin, Leucin, Methionin, Asparagin, Prolin, Glutamin, Serin, Threonin oder Valin ist.

9. Das Transportprotein nach Anspruch 7, wobei die substituierte Aminosäure Alanin, Cystein oder Valin ist.

10. Das Transportprotein nach Anspruch 1, wobei die Aminosäure der Botulinum Neurotoxin Typ A Proteinsequenzen in Position 1252 durch Tyrosin ersetzt wird oder in Position 1253 durch Lysin ersetzt wird.

11. Das Transportprotein nach Anspruch 1, wobei zwei oder drei Aminosäuren ausgewählt aus den Positionen 1117, 1252, 1253, 1270, 1278 bis 1279 der Botulinum Neurotoxin Typ A Proteinsequenzen entfernt oder substituiert werden, vorzugsweise Positionen 1117/1252, 1117/1253, 1117/1278, 1117/1279, 1117/1252/1253, besonders bevorzugt Y1117A/F1252Y, Y1117A/H1253K, Y1117A/L1278H, Y1117A/G1279N, Y1117C/F1252Y, Y1117C/H1253K, Y1117C/L1278H, Y1117C/G1279N, Y1117V/F1252Y, Y1117V/H1253K, Y1117V/L1278H, Y1117V/G1279N, Y1117A/F1252Y/H1253K, Y1117C/F1252Y/H1253K und Y1117V/F1252/H1253K.

12. Eine Zusammensetzung, enthaltend ein Transportprotein nach einem der Ansprüche 1 bis 11, und wenigstens ein intervenierendes Molekül, wobei das intervenierende Molekül entweder ein kleines organisches Molekül, ein Peptid oder ein Protein umfasst.

13. Die Zusammensetzung nach Anspruch 12, wobei das intervenierende Molekül durch eine Peptid-Bindung, Ester-Bindung, Ether-Bindung, Sulfid-Bindung, Disulfid-Bindung oder Kohlenstoff-Kohlenstoff-Bindung kovalent gebunden ist.

14. Die Zusammensetzung nach Anspruch 12, wobei das kleine organische Molekül ein Virustatikum, Zytostatikum, Antibiotikum oder ein Immunoglobulin ist.

15. Die Zusammensetzung nach Anspruch 12, wobei das Protein eine Protease umfasst.

16. Die Zusammensetzung nach Anspruch 15, wobei die Protease ein Neurotoxin Protein eines *Clostridium botulinum* Typs A, B, C₁, D, E, F und G umfasst.

17. Die Zusammensetzung nach Anspruch 15, wobei die Protease ein proteolytisch aktives Fragment enthält, welches von dem Protein aus *Clostridium botulinum* des Typs A, B, C₁, D, E, F und G abgeleitet ist.

18. Die Zusammensetzung nach Anspruch 17, dadurch charakterisiert, dass die Protease die Sequenz His-Glu-Leu-Xaa-His-(Xaa)₃₃₋₃₅-Glu-(Xaa)₈₄₋₉₀-Glu-(Xaa)₁₁-Arg-Xaa-Xaa-Tyr enthält, wobei Xaa irgendeine Aminosäure sein kann, und wobei die Protease spezifische Substrate innerhalb des cholinergen Motoneurons spaltet.

19. Die Zusammensetzung nach Anspruch 18 wobei die Substrate ausgewählt sind aus Proteinen, die in der Freisetzung von Neurotransmittern in peripheren Nerven involviert sind, und Proteinen, die zu katalytischen Reaktionen innerhalb der Nervenzelle fähig sind.

20. Die Zusammensetzung nach Anspruch 19, wobei die Protease und das Transportprotein kovalent durch eine Aminosäuresequenz verbunden sind, die von einer Endopeptidase spezifisch erkannt und gespalten wird.

21. Die Zusammensetzung nach Anspruch 20, wobei nach Spaltung durch die Endopeptidase eine Disulfid-Brücke die Protease und das Transportprotein miteinander verbindet, welches wiederum zur Entstehung eines aktiven Holotoxins führt.

22. Eine pharmazeutische Zusammensetzung, die das Transportprotein nach einem der Ansprüche 1 bis 11 oder die Zusammensetzung nach einem der Ansprüche 12 bis 21 enthält, sowie optional einen pharmazeutisch akzeptablen Träger, Verdünnungsmittel und /oder Additiv.

23. Pharmazeutische Zusammensetzung nach Anspruch 22 zur Behandlung einer Störung oder Erkrankung, für welche eine Therapie mit Botulinus Neurotoxin indiziert ist.

24. Pharmazeutische Zusammensetzung nach Anspruch 23, wobei die Störung oder Erkrankung eine der folgenden ist: Hemifacialspasmus, spasmodischer Torticollis, Spastizitäten, Dystonien, Migräne, Schmerzen, Erkrankungen der Hals- und Lendenwirbelsäule, Strabismus, Hypersalivation und depressive Erkrankungen.

25. Eine kosmetische Zusammensetzung, die das Transportprotein nach einem der Ansprüche 1 bis 11 oder die Zusammensetzung nach einem der Ansprüche 12 bis 21 enthält, sowie optional einen pharmazeutisch akzeptablen Träger, Verdünnungsmittel und /oder Additiv.

26. Verwendung einer kosmetischen Zusammensetzung nach Anspruch 25 als kosmetisches Erzengnis, wobei die kosmetischen Indikationen Hyperhydrose und stark ausgeprägte Gesichtsfalten sind.

27. Ein Verfahren zur Herstellung eines Transportproteins nach einem der Ansprüche 1 bis 11 oder einer Zusammensetzung nach einem der Ansprüche 12 bis 21 durch Rekombination gemäß bekannter Verfahren.

28. Eine Wirtszelle, die einen rekombinanten Expressionsvektor enthält, wobei der Expressionsvektor ein Transportprotein gemäß einem der Ansprüche 1 bis 11 oder eine Zusammensetzung nach einem der Ansprüche 12 bis 23 kodiert.

29. Die Wirtszelle nach Anspruch 28, wobei die Wirtszelle eine Zelle von *Escherichia coli, Saccharomyces cerevisiae, Pichia pastoris* oder *Bacillus megaterium* sein kann.

30. Ein Expressionsvektor, wobei der Vektor eine Nukleinsäure umfasst, die ein Transportprotein nach einem der Ansprüche 1 bis 11 oder eine Zusammensetzung nach einem der Ansprüche 12 bis 21 kodiert.

## Claims

1. Transport protein, obtainable by modification of the heavy chain of the neurotoxin formed by *Clostridium botulinum,* wherein the neurotoxin is botulinum neurotoxin type A (BoNT/A) and wherein the transport protein binds to nerve cells with higher affinity than the native neurotoxin, wherein the binding activity is determined in the synaptosome-assay, and wherein at least one amino acid in the positions 1117, 1252, 1253, 1270, 1278 to 1279 of the botulinum neurotoxin type A protein sequences is removed or substituted, either by an amino acid which is naturally occurring or which is of non-natural origin.

2. Transport protein, obtainable by modification of the heavy chain of the neurotoxin formed by *Clostridium botulinum* neurotoxin type A (BoNT/A), wherein the transport protein binds to nerve cells with higher affinity than the native neurotoxin, wherein the binding activity is determined in the synaptosome-assay, and wherein the amino acids 1092 to 1296 of *Clostridium botulinum* neurotoxin type A, which contain the ganglioside-binding domain, are substituted with the subsequent sequences:
*Clostridium botulinum* neurotoxin type B protein, amino acids 1079 to 1291, or
*Clostridium botulinum* neurotoxin type C₁ protein, amino acids 1093 to 1291, or
H_{cc}-domain of *Clostridium tetani* neurotoxin proteins.

3. Transport protein, obtainable by modification of the heavy chain of the neurotoxin formed by *Clostridium botulinum* neurotoxin type A (BoNT/A), wherein the transport protein binds to nerve cells with higher affinity than the native neurotoxin, wherein the binding activity is determined in the synaptosome-assay, and wherein the complete H_{c}-fragment of BoNT/A (amino acids 867 to 1296) is substituted by the H_{c}-fragment of BoNT/B (amino acids 866 to 1291).

4. The transport protein of claim 1, 2 or 3, wherein the transport protein specifically binds to nerve cells and enters the cells by endocytosis.

5. The transport protein of claim 1, 2 or 3, wherein the transport protein binds specifically to complex gangliosides of cholinergic motor neurons, localised in the plasma membrane, preferably to GT1b.

6. The transport protein of any one of claims 1 to 5, wherein the transport protein exhibits an affinity which is at least 15% higher than the native neurotoxin, preferably at least 50%, particularly preferably at least 80% and in particular at least 90%.

7. The transport protein of claim 1, wherein the amino acid in position 1117 of the botulinum neurotoxin type A protein sequences is removed or substituted, either by an amino acid which is naturally occurring or which is of non-natural origin.

8. The transport protein of claim 7, wherein the substituted amino acid is either alanine, cysteine, glutamate, phenyl alanine, isoleucine, leucine, methionine, asparagine, proline, glutamine, serine, threonine or valine.

9. The transport protein of claim 7, wherein the substituted amino acid is alanine, cysteine or valine.

10. The transport protein of claim 1, wherein the amino acid in position 1252 of the botulinum neurotoxin type A protein sequences is substituted by a tyrosine or in position 1253 by a lysine.

11. The transport protein of claim 1, wherein two or three amino acids selected from positions 1117, 1252, 1253, 1270, 1278 to 1279 of the botulinum neurotoxin type A protein sequences are removed or substituted, preferably positions 1117/1252, 1117/1253, 1117/1278, 1117/1279, 1117/1252/1253, particularly preferably Y1117A/F1252Y, Y1117A/H1253K, Y1117A/L1278H, Y1117A/G1279N, Y1117C/F1252Y, Y1117C/H1253K, Y1117C/L1278H, Y1117C/G1279N, Y1117V/F1252Y, Y1117V/H1253K, Y1117V/L1278H, Y1117V/G1279N, Y1117A/F1252Y/H1253K, Y1117C/F1252Y/H1253K and Y1117V/F1252/H1253K.

12. Composition containing a transport protein of any one of claims 1 to 11 and at least one intervening molecule, wherein the intervening molecule either comprises a small organic molecule, a peptide or a protein.

13. The composition of claim 12, wherein the intervening molecule is covalently bonded by a peptide bond, ester-bond, ether-bond, sulphide-bond, disulphide-bond or carbon-carbon-bond.

14. The composition of claim 12, wherein the small organic molecule is a virustatic, a cytostatic, an antibiotic or an immunoglobulin.

15. The composition of claim 12, wherein the protein comprises a protease.

16. The composition of claim 15, wherein the protease comprises a neurotoxin protein of a *Clostridium botulinum* type A, B, C₁, D, E, F and G.

17. The composition of claim 15, wherein the protease contains a proteolytically active fragment, which is derived from the protein of *Clostridium botulinum* type A, B, C₁, D, E, F and G.

18. The composition of claim 17, **characterized in that** it contains the sequence His-Glu-Leu-Xaa-His-(Xaa)₃₃₋₃₅-Glu-(Xaa)₈₄₋₉₀-Glu-(Xaa)₁₁-Arg-Xaa-Xaa-Tyr, wherein Xaa may be any amino acid, and wherein the protease cleaves specific substrates within the cholinergic motor neuron.

19. The composition of claim 18, wherein the substrates are selected from proteins involved in the release of neurotransmitters in peripheral nerves and from proteins capable of catalytic reactions within the nerve cell.

20. The composition of claim 19, wherein the protease and the transport protein are covalently bonded by an amino acid sequence, which is specifically recognised and cleaved by an endopeptidase.

21. The composition of claim 20, wherein after cleavage by the endopeptidase a disulphide-bridge interlinks the protease and the transport protein, which, in turn, results in the formation of an active holotoxin.

22. Pharmaceutical composition containing the transport protein of any one of claims 1 to 11 or the composition of any one of claims 12 to 21, as well as optionally a pharmaceutically acceptable carrier, diluent and/or additive.

23. The pharmaceutical composition of claim 22 for treating a disorder or disease for which a therapy with botulinum neurotoxin is indicated.

24. The pharmaceutical composition of claim 23, wherein the disorder or disease is one of the following: hemi-facial spasm, spasmodic torticollis, spasticities, dystonias, migraine, pain, disorders of the neck- and lumbar vertebral column, strabism, hypersalivation and depressive diseases.

25. Cosmetic composition containing the transport protein of any one of claims 1 to 11 or the composition of any one of claims 12 to 21, as well as optionally a pharmaceutically acceptable carrier, diluent and/or additive.

26. Use of a cosmetic composition of claim 25 as cosmetic product, wherein the cosmetic indications are hyperhidrosis and pronounced facial wrinkles.

27. Process for the production of a transport protein of any one of claims 1 to 11 or of a composition of any one of claims 12 to 21 by recombination according to known methods.

28. Host cell containing a recombinant expression vector, wherein the expression vector encodes a transport protein of any one of claims 1 to 11 or a composition of any one of claims 12 to 23.

29. The host cell of claim 28, wherein the host cell may be a cell of *Escherichia coli, Saccharomyces cerevisiae, Pichia pastoris* or *Bacillus megaterium.*

30. An expression vector, wherein the vector comprises a nucleic acid encoding a transport protein according to any one of claims 1 to 11 or a composition according to any one of claims 12 to 21.

## Revendications

1. Protéine de transport, pouvant être obtenue par modification de la chaîne lourde de la neurotoxine formée à partir de *Clostridium botulinum,* dans laquelle la neurotoxine est la neurotoxine type A de botulinum (BoNT/A) et dans laquelle la protéine de transport se lie à des cellules nerveuses avec une affinité plus élevée que la neurotoxine native, l'activité de liaison étant déterminée dans l'essai sur synaptosomes, et tandis qu'au moins un acide aminé dans les positions 1117, 1252, 1253, 1270, 1278 à 1279 des séquences protéiques de neurotoxine type A de botulinum est éliminé ou est substitué par un acide aminé qui soit est naturel, soit n'est pas d'origine naturelle.

2. Protéine de transport, pouvant être obtenue par modification de la chaîne lourde de la neurotoxine type A de *Clostridium botulinum,* (BoNT/A), dans laquelle la protéine de transport se lie à des cellules nerveuses avec une affinité plus élevée que la neurotoxine native, tandis que l'activité de liaison est déterminée par l'essai sur synaptosomes, et tandis que les acides aminés 1092 à 1296 de la neurotoxine type A de *Clostridium botulinum,* qui contiennent les domaines de liaison aux gangliosides, sont remplacés par les séquences suivantes:
protéine de neurotoxine type B de *Clostridium botulinum,* acides aminés 1079 à 1291, ou
protéine de neurotoxine type C₁ de *Clostridium botulinum,* acides aminés 1093 à 1291, ou
domaine H_{CC} de la protéine de neurotoxine de *Clostridium tetani.*

3. Protéine de transport, pouvant être obtenue par modification de la chaîne lourde de la neurotoxine type A de *Clostridium botulinum* (BoNT/A), dans laquelle la protéine de transport se lie aux cellules nerveuses avec une affinité supérieure à celle de la neurotoxine native, tandis que l'activité de liaison est déterminée dans l'essai sur synaptosomes, et tandis que le fragment H_{C} de BoNT/A complet (acides aminés 867 à 1296) est remplacé par le fragment H_{C} de BoNT/B (acides aminés 866 à 1291).

4. Protéine de transport selon la revendication 1, 2 ou 3, dans laquelle la protéine de transport se lie spécifiquement à des cellules nerveuses et est captée par endocytose par les cellules.

5. Protéine de transport selon la revendication 1, 2 ou 3, dans laquelle la protéine de transport se lie spécifiquement à des gangliosides complexes de neurones moteurs cholinergiques, qui sont localisés dans la membrane plasmatique, de préférence à GT1b.

6. Protéine de transport selon l'une des revendications 1 à 5, dans laquelle la protéine de transport présente une affinité d'au moins 15% supérieure à la neurotoxine native, de préférence d'au moins 50%, particulièrement préférablement d'au moins 80%, tout particulièrement d'au moins 90%.

7. Protéine de transport selon la revendication 1, dans laquelle l'acide aminé en position 1117 des séquences protéiques de neurotoxine type A de botulinum est éliminé ou est substitué par un acide aminé qui soit est naturel, soit n'est pas d'origine naturelle.

8. Protéine de transport selon la revendication 7, dans laquelle l'acide aminé substitué est l'alanine, la cystéine, le glutamate, la phénylalanine, l'isoleucine, la leucine, la méthionine, l'asparagine, la proline, la glutamine, la sérine, la thréonine ou la valine.

9. Protéine de transport selon la revendication 7, dans laquelle l'acide aminé substitué est l'alanine, la cystéine ou la valine.

10. Protéine de transport selon la revendication 1, dans laquelle l'acide aminé des séquences protéiques de neurotoxine type A de botulinum dans la position 1252 est remplacé par la tyrosine ou dans la position 1253 est remplacé par la lysine.

11. Protéine de transport selon la revendication 1, dans laquelle deux ou trois acides aminés choisis sur les positions 1117,1252,1253,1270,1278 à 1279 des séquences protéiques de neurotoxine type A de botulinum sont éliminés ou sont remplacés, de préférence les positions 1117/1252, 1117/1253, 1117/1278, 1117/1279, 1117/1252/1253, particulièrement préférablement Y1117A/F1252Y, Y1117A/H1253K, Y1117A/L1278H, Y1117A/G1279N, Y1117C/F1252Y, Y1117C/H1253K, Y1117C/L1278H, Y1117C/G1279N, Y1117V/F1252Y, Y1117V/H1253K, Y1117V/L1278H, Y1117V/G1279N, Y1117A/F1252Y/H1253K, Y1117C/F1252Y/H1253K et Y1117V/F1252/H1253K.

12. Composition contenant une protéine de transport selon l'une des revendications 1 à 11, et au moins une molécule intervenante, tandis que la molécule intervenante englobe une petite molécule organique, un peptide ou une protéine.

13. Composition selon la revendication 12, dans laquelle la molécule intervenante est liée par covalence par l'intermédiaire d'une liaison peptidique, d'une liaison ester, d'une liaison éther, d'une liaison sulfure, d'une liaison disulfure ou d'une liaison carbone-carbone.

14. Composition selon la revendication 12, dans laquelle la petite molécule organique est un agent virostatique, cytostatique, antibiotique, ou une immunoglobuline.

15. Composition selon la revendication 12, dans laquelle la protéine comporte une protéase.

16. Composition selon la revendication 15, dans laquelle la protéase comporte une protéine de neurotoxine d'un *Clostridium botulinum* de type A, B, C₁, D, E, F et G.

17. Composition selon la revendication 15, dans laquelle la protéase contient un fragment actif du point de vue protéolytique, qui provient de la protéine de *Clostridium botulinum* du type A, B, C₁, D, E, F et G.

18. Composition selon la revendication 17, **caractérisée en ce que** la protéase contient la séquence His-Glu-Leu-Xaa-His-(Xaa)₃₃₋₃₅-Glu-(Xaa)₈₄₋₉₀-Glu-(Xaa)₁₁-Arg-Xaa-Xaa-Tyr, tandis que Xaa peut être un acide aminé quelconque, et tandis que la protéase coupe des substrats spécifiques à l'intérieur du neurone moteur cholinergique.

19. Composition selon la revendication 18, dans laquelle les substrats sont choisis parmi les protéines qui sont impliquées dans la libération des neurotransmetteurs dans les nerfs périphériques, et les protéines qui sont aptes à donner des réactions catalytiques à l'intérieur de la cellule nerveuse.

20. Composition selon la revendication 19, dans laquelle la protéase et la protéine de transport sont liées par covalence par l'intermédiaire d'une séquence d'acides aminés, qui est reconnue et coupée spécifiquement par une endopeptidase.

21. Composition selon la revendication 20, dans laquelle après coupure par l'intermédiaire de l'endopeptidase, un pont disulfure relie entre elles la protéase et la protéine de transport, qui à son tour conduit à la formation d'une holotoxine active.

22. Composition pharmaceutique, qui contient la protéine de transport selon l'une des revendications 1 à 11 ou la composition selon l'une des revendications 12 à 21, ainsi que, en option, un support, un agent diluant et/ou un additif pharmaceutiquement acceptables.

23. Composition pharmaceutique selon la revendication 22 pour le traitement d'une perturbation ou d'une affection, pour laquelle une thérapie avec une neurotoxine de botulinum est indiquée.

24. Composition pharmaceutique selon la revendication 23, dans laquelle la perturbation ou l'affection est l'une des suivantes: spasme hémifacial, torticolis spasmodique, spasticité, dystonie, migraine, maux de tête, affections des vertèbres cervicales et lombaires, strabisme, hypersalivation et maladies dépressives.

25. Composition cosmétique, qui contient la protéine de transport selon l'une des revendications 1 à 11 ou la composition selon l'une des revendications 12 à 21, ainsi que, en option, un support, un agent diluant et/ou un additif pharmaceutiquement acceptables.

26. Utilisation d'une composition cosmétique selon la revendication 25 comme produit cosmétique, tandis que les indications cosmétiques sont 1 hyperhydrose et les rides faciales fortement prononcées.

27. Procédé pour la préparation d'une protéine de transport selon l'une des revendications 1 à 11 ou d'une composition selon l'une des revendications 12 à 21 par recombinaison selon des procédés connus.

28. Cellule hôte, qui contient un vecteur d'expression recombinant, tandis que le vecteur d'expression code pour une protéine de transport selon l'une des revendications 1 à 11 ou une composition selon l'une des revendications 12 à 23.

29. Cellule hôte selon la revendication 28, tandis que la cellule hôte peut être une cellule de *Escherichia coli, Saccharomyces cerevisiae, Pichia pastoris* ou *Bacillus megaterium.*

30. Vecteur d'expression, dans lequel le vecteur comprend un acide nucléique qui code pour une protéine de transport selon l'une des revendications 1 à 11 ou pour une composition selon l'une des revendications 12 à 21.
